# EUROPÄISCHE PATENTANMELDUNG

(11) **EP 1 709 926 A1**
(43) Veröffentlichungstag der Anmeldung: **11.10.2006**
(21) Anmeldenummer: 06006550.5
(22) Anmeldetag: 29.03.2006
(51) Int. Cl.: A61B 19/02, A61L 2/26, A61B 19/00

(54) **Siebkorb für chirurgische Instrumente**

(30) Priorität: 08.04.2005 DE 102005017680
(71) Anmelder: Aesculap AG & Co. KG, 78532 Tuttlingen (DE)
(72) Erfinder: Jakab, Mariana, Dipl.-Ing., 78532 Tuttlingen (DE); Schuster, Stefan, Dipl.-Ing., 78048 Villingen-Schwenningen (DE)
(74) Vertreter: Hoeger, Stellrecht & Partner Patentanwälte

(57) **Zusammenfassung**

Siebkorb (10) zur Aufnahme chirurgischer oder zahnchirurgischer Instrumente oder Geräte mit einem Boden (12) und Seitenwänden (16,18), wobei der Boden und/oder die Seitenwände eine Vielzahl von Durchbrechungen (20,32,40) aufweisen, so zu verbessern, daß Instrumente und Geräte optimal gereinigt werden können, wird vorgeschlagen, daß der Boden und/oder mindestens eine Seitenwand mindestens eine, von einer Form und/oder Größe der Durchbrechungen (40) abweichende Form und/oder Größe aufweisende Lagerelementaufnahme (42) zum Festlegen eines Lagerelements (66) zum Haltern mindestens eines chirurgischen oder zahnchirurgischen Instruments oder Geräts aufweist.

## Beschreibung

Die vorliegende Erfindung betrifft einen Siebkorb zur Aufnahme chirurgischer oder zahnchirurgischer Instrumente oder Geräte mit einem Boden und Seitenwänden, wobei der Boden und/oder die Seitenwände eine Vielzahl von Durchbrechungen aufweisen.

Siebkörbe der eingangs beschriebenen Art werden üblicherweise dazu verwendet, um darin aufgenommene chirurgische oder zahnchirurgische Instrumente und/oder Geräte in eine Spülvorrichtung einzubringen, beispielsweise in eine Spülmaschine. Die Durchbrechungen dienen einerseits dazu, daß ein Reinigungsfluid auch durch den Boden und/oder die Seitenwände an die im Siebkorb befindlichen Instrumente und Geräte herankommen kann, zum anderen dienen die Durchbrechungen dazu, daß nach Abschluß der Reinigung die verwendete Reinigungsflüssigkeit durch die Durchbrechungen hindurch abfließen kann, wodurch Flüssigkeitsansammlungen im Bereich der Instrumente oder Geräte im Innern des Siebkorbs vermieden werden. Ferner dienen Siebkörbe auch der dauerhaften Lagerung von Instrumenten oder Geräten und können hierfür zusätzlich in einen vollständig geschlossenen Behälter eingebracht werden, beispielsweise in einen Sterilcontainer. Des weiteren dienen sie der Bereitstellung darin gelagerter Instrumente und/oder Geräte für einen Operateur oder Zahnarzt.

Siebkörbe der eingangs beschriebenen Art sind üblicherweise aus einem Metall hergestellt. Um Beschädigungen der im Siebkorb aufgenommenen Instrumente oder Geräte zu vermeiden, ist es bekannt, beispielsweise Silikonmatten in die Siebkörbe einzulegen, um die Instrumente oder Geräte zu schützen.

Allerdings können Instrumente oder Geräte, unabhängig davon, ob zusätzlich Silikonmatten verwendet werden oder nicht, im Siebkorb verrutschen und im schlimmsten Fall so ungünstig übereinanderliegen, daß sie bei der Reinigung nicht von allen Seiten von einer Reinigungsflüssigkeit erreicht werden können.

Es ist daher Aufgabe der vorliegenden Erfindung, einen Siebkorb der eingangs beschriebenen Art so zu verbessern, daß Instrumente und Geräte optimal geschützt und gereinigt werden können.

Diese Aufgabe wird bei einem Siebkorb der eingangs beschriebenen Art erfindungsgemäß dadurch gelöst, daß der Boden und/oder mindestens eine Seitenwand mindestens eine, eine von einer Form und/oder Größe der Durchbrechungen abweichende Form und/oder Größe aufweisende Lagerelementaufnahme zum Festlegen eines Lagerelements zum Halten mindestens eines chirurgischen oder zahnchirurgischen Instruments oder Geräts aufweist.

Mittels der mindestens einen Lagerelementaufnahme kann ein Lagerelement am Boden und/oder einer Seitenwand festgelegt werden, wobei das Lagerelement vorzugsweise dazu dient, ein chirurgisches oder zahnchirurgisches Instrument oder Gerät in einer definierten Position zu halten, und zwar nicht nur während einer Reinigung, sondern auch während eines nachfolgenden Sterilisierprozesses, dem die Instrumente und Geräte unterworfen werden, sowie auch noch danach, wenn die Instrumente und Geräte im Siebkorb oder in einem Container, in den der Siebkorb eingebracht werden kann, gelagert werden. Insbesondere kann die Lagerelementaufnahme als Durchbrechung ausgebildet sein, sodaß eine Abschattung für eine Reinigungsflüssigkeit durch verbleibende Teile des Bodens oder einer Seitenwand minimiert wird, wenn kein Lagerelement mit der Lagerelementaufnahme verbunden ist.

Vorteilhaft ist es, wenn die Durchbrechungen des Bodens in einem regelmäßigen Durchbrechungsmuster angeordnet sind. Auf diese Weise wird sichergestellt, daß eine von allen Durchbrechungen gebildete Durchtrittsfläche insgesamt ausreichend groß ist, damit eine Reinigungsflüssigkeit alle im Siebkorb aufgenommenen Instrumente oder Geräte erreichen kann. Ferner wird die Herstellung des Siebkorbs so besonders einfach.

Vorzugsweise weist das Durchbrechungsmuster eine zwei- drei, vier- oder sechszählige Symmetrie auf. Dies bedeutet, daß das Durchbrechungsmuster durch Drehung um 180°, 120°, 90° oder 60° in sich selbst überführt werden kann.

Besonders einfach wird der Aufbau des Siebkorbs, wenn die Durchbrechungen in Reihen und Spalten angeordnet sind.

Der Aufbau und die Herstellung des Siebkorbs vereinfachen sich weiter, wenn alle Durchbrechungen identisch ausgebildet sind.

Vorzugsweise sind die Durchbrechungen eckig ausgebildet, wodurch eine maximale Durchtrittsfläche ausgebildet werden kann.

Um eine ausreichende Stabilität des Siebkorbs zu gewährleisten, ist es günstig, wenn zwischen den Durchbrechungen des Bodens angeordnete Stege vorgesehen sind.

Gemäß einer bevorzugten Ausführungsform der Erfindung kann vorgesehen sein, daß die Stege drahtförmig ausgebildet und netzartig miteinander verbunden sind. Dies gewährleistet eine ausreichende Stabilität des Siebkorbs. Auch eine Herstellung des Siebkorbs mit derartigen Stegen ist mit einem begrenzten Aufwand möglich.

Der Siebkorb läßt sich beispielsweise durch Ausstanzen herstellen, wenn die Stege einstückig ausgebildet sind als Streifen, die beim Einarbeiten der Durchbrechungen in einen plattenförmigen Boden zwischen den Durchbrechungen stehenbleiben. Alternativ wäre es auch denkbar, die Durchbrechungen zum Beispiel durch Erodieren oder Laserschneiden herzustellen.

Günstig ist es, wenn mehrere in einem regelmäßigen Lagerelementaufnahmenmuster angeordnete Lagerelementaufnahmen vorgesehen sind. Zum einen wird dadurch die Herstellung des Siebkorbs vereinfacht, zum anderen bietet eine solche Ausgestaltung die Möglichkeit, Lagerelemente variabel am Siebkorb festzulegen. Insbesondere kann eine Anordnung von Lagerelementen individuell angepaßt oder aber auch geändert werden.

Vorzugsweise weist das Lagerelementaufnahmenmuster eine zwei-, drei-, vier- oder sechszählige Symmetrie auf. Vorzugsweise entspricht eine Symmetrie des Lagerelementaufnahmenmusters der des Durchbrechungsmusters.

Vorteilhaft ist es, wenn das Lagerelementaufnahmenmuster ein das Durchbrechungsmuster unterbrechendes Muster ist, wenn das Lagerelementaufnahmenmuster den Boden und/oder eine Seitenwand in einzelne Flächenelemente aufteilt und wenn die Flächenelemente nur Durchbrechungen aufweisen. Zwischen den Flächenelementen können beispielsweise Lagerelementaufnahmen angeordnet sein, sodaß die Lagerelementaufnahmen quasi ein übergeordnetes Muster oder Übermuster bilden, wie es insbesondere hergestellt werden könnte in einem Boden oder einer Seitenwand, der oder die zunächst vollständig nur mit Durchbrechungen versehen ist, wobei nachträglich Lagerelementaufnahmen angeordnet werden entsprechend dem Lagerelementaufnahmenmuster, beispielsweise durch Entfernen von einzelnen Durchbrechungen miteinander verbindende Bodenbereiche, zum Beispiel durch Entfernen von Stegen. Insbesondere dann, wenn die mindestens eine Lagerelementaufnahme größer ist als eine Durchbrechung, kann eine Reinigungsflüssigkeit noch besser in den Siebkorb eindringen oder aus diesem ablaufen. Zudem kann ein Lagerelement auf einfache Weise mit dem Boden verbunden werden, beispielsweise durch Einschieben in die Lagerelementaufnahme. Die Flächenelemente nur mit Durchbrechungen und nicht mit Lagerelementaufnahmen zu versehen hat den Vorteil, daß beispielsweise ein Boden oder eine Seitenwand des Siebkorbs durch Zusammenfügen aus derartigen Flächenelementen hergestellt werden kann, beispielsweise durch Verschweißen derartiger Flächenelemente.

Der Aufbau des Siebkorbs wird besonders einfach, wenn die Durchbrechungen des Flächenelements entsprechend dem Durchbrechungsmuster angeordnet sind. Dadurch kann im Bereich des Flächenelements eine Abschattung für eine Reinigungsflüssigkeit minimiert werden.

Vorteilhaft ist es, wenn eine Form der Flächenelemente einer Form der Durchbrechungen geometrisch ähnlich ist. Auf diese Weise läßt sich bevorzugt ein zwei- oder vierzähliges, aber auch ein drei- oder sechszähliges Muster durch entsprechende Anordnung der Flächenelemente ausbilden. Beispielsweise können die Flächenelemente quadratisch oder rechteckig geformt sein. Denkbar wären auch andere vieleckige Formen, insbesondere Dreiecke oder Sechsecke.

Um den Herstellungsaufwand für den Siebkorb zu vereinfachen, ist es günstig, wenn die Flächenelemente regelmäßig angeordnet sind und wenn die Flächenelemente mehrere regelmäßig angeordnete Durchbrechungen aufweisen. Eine derartige Ausgestaltung des Siebkorbs ermöglicht es, mit einer minimalen Anzahl an Werkzeugen den Siebkorb herzustellen. Beispielsweise können die Durchbrechungen und die diese aufweisenden Flächenelemente mit einem, höchstens zwei Stanzwerkzeugen aus einem Blech oder eine Platte ausgestanzt werden.

Vorteilhafterweise wird die mindestens eine Lagerelementaufnahme seitlich durch mindestens zwei Flächenelemente begrenzt. Die mindestens zwei Flächenelemente bilden somit eine abschnittsweise Begrenzung, man könnte auch sagen einen Teil eines Rahmens, der Lagerelementaufnahme.

Eine besonders flexible Anordnungsmöglichkeit für Lagerelemente im Siebkorb ist möglich, wenn das mindestens eine Flächenelement 16, jeweils in vier Reihen und Spalten angeordnete Durchbrechungen aufweist. Ein Boden oder eine Seitenwand kann aus einer Vielzahl dieser Flächenelemente zusammengesetzt werden.

Die Herstellung des Siebkorbs vereinfacht sich weiter, wenn alle Flächenelemente identisch ausgebildet sind.

Grundsätzlich wäre es denkbar, die Flächenelemente direkt miteinander zu verbinden. Vorzugsweise sind jedoch die Flächenelemente über Kreuzungselemente miteinander verbunden. Die Kreuzungselemente ermöglichen es, einen Abstand zwischen zwei Flächenelementen in gewünschter Weise vorzusehen. Ferner bieten die Kreuzungselemente die eine ausreichend große Kontaktfläche und damit die Möglichkeit, andere Teile, beispielsweise besondere Formen von Lagerelementen, am Boden oder einer Seitenwand zu befestigen, beispielsweise durch Punktschweißen. Der erfindungsgemäß vorgeschlagene Siebkorb bietet somit die Möglichkeit, zusätzliche Lagerungselemente durch Anschweißen an die Kreuzungselemente, durch Verschrauben mittels durch die Durchbrechungen durchgeführter Schrauben oder durch anclipsen an die Durchbrechungen oder Lagerelementaufnahmen mit dem Boden und/oder einer Seitenwand des Siebkorbs zu verbinden.

Vorzugsweise wird die mindestens eine Lagerelementaufnahme durch mindestens ein Kreuzungselement begrenzt. Diese Ausgestaltung ermöglicht es, Lagerelementaufnahmen kreuzförmig oder sternförmig, ausgehend von einem Kreuzungselement, anzuordnen.

Auf einfache Weise kann ein Lagerelementaufnahmenmuster generiert werden, wenn die mindestens eine Lagerelementaufnahme von zwei Flächenelementen und von zwei Kreuzungselementen begrenzt wird.

Grundsätzlich wäre es denkbar, daß das mindestens eine Kreuzungselement Seiten oder seitliche Stege der Flächenelemente miteinander verbindet. Um jedoch eine Verletzungsgefahr durch eventuell scharfkantige Ecken der Flächenelemente zu minimieren, ist es günstig, wenn das mindestens eine Kreuzungselement Ecken der Flächenelemente miteinander verbindet.

Um ein Durchbrechungsmuster oder ein Lagerelementaufnahmenmuster mit einer zwei- oder vierzähligen Symmetrie zu erhalten, ist es vorteilhaft, wenn das mindestens eine Kreuzungselement vier Flächenelemente miteinander verbindet.

Bei Siebkörben mit gitternetzartig angeordneten Stegen, die die Durchbrechungen begrenzen, stehen nur sehr kleine Flächen zur Verfügung, um beispielsweise durch Punktschweißen weitere tragende Elemente am Siebkorb festzulegen. Es ist daher von Vorteil, wenn das mindestens eine Kreuzungselement eine Fläche aufweist, die größer ist als eine von zwei sich kreuzenden, die Durchbrechungen trennenden Stege gebildete Kreuzungsfläche. Die Kreuzungselemente bieten so die Möglichkeit, weitere Elemente auf einfache Weise am Siebkorb festzulegen, wobei eine Breite der die Durchbrechungen trennenden Stege minimal klein gehalten werden kann, um eine Abschattung für eine Reinigungsflüssigkeit möglichst gering zu halten.

Vorteilhafterweise ist die vom mindestens einen Kreuzungselement definierte Fläche mindestens zehnmal größer als die Kreuzungsfläche, vorzugsweise sogar sechzehnmal. Dadurch wird sowohl die Anlagefläche für ein weiteres Element, welches am Siebkorb festgelegt werden soll als auch eine Befestigungsfläche vergrößert, wodurch ein zusätzliches Element am Siebkorb dauerhaft deutlich besser gehalten wird.

Gemäß einer bevorzugten Ausführungsform der Erfindung kann vorgesehen sein, daß die vom mindestens einen Kreuzungselement definierte Fläche in etwa einer Querschnittsfläche einer Durchbrechung entspricht. Ist die Durchbrechung ausreichend groß, um Reinigungsflüssigkeit durchzulassen und um eine Abschattung zu minimieren, dann reicht auch die vom Kreuzungselement definierte Fläche aus, um als Anlagefläche oder Befestigungsfläche für ein zusätzliches Element, beispielsweise ein weiteres Lagerungselement, des Siebkorbs zu dienen.

Günstigerweise grenzen mindestens zwei Lagerelementaufnahmen aneinander an. Beispielsweise können diese in einer Reihe angeordnet sein, sodaß sich Lagerelemente in einer Reihe anordnen lassen. Beispielsweise können eine Längsrichtung definierende Lagerelementaufnahmen mit ihren Stirnseiten aneinandergrenzen.

Vorzugsweise ist die mindestens eine Lagerelementaufnahme in Form eines Langlochs ausgebildet. Ein Langloch ist besonders einfach herzustellen und eignet sich hervorragend, um ein Lagerelement aufzunehmen und festzulegen.

Eine ausreichende Stabilität der mindestens einen Lagerelementaufnahme ist gewährleistet, wenn diese von zwei parallelen Längsstegen und zwei die Längsstege verbindenden Querstegen begrenzt wird.

Vorteilhaft ist es, wenn die mindestens eine Lagerelementaufnahme von zwei parallel zueinander verlaufenden Längsstegen und zwei die Längsstege verbindenden Querstegen begrenzt wird. Auf diese Weise kann eine ausreichende Stabilität der Lagerelementaufnahme sichergestellt werden.

Um eine Verletzungsgefahr für eine Bedienperson durch den Siebkorb zu minimieren, ist es günstig, wenn die zwei Querstege der mindestens einen Lagerelementaufnahme aufeinander zu weisend konkav gekrümmt sind.

Ein Aufbau des Siebkorbs wird weiter vereinfacht, wenn die Querstege halbkreisförmig ausgebildet sind. Günstigerweise bilden mindestens zwei Querstege ein Kreuzungselement. Auf diese Weise kann der Aufbau des Siebkorbs weiter vereinfacht werden. Zudem ist es so möglich, zwei Lagerelementaufnahmen über ein Kreuzungselement miteinander zu verbinden.

Vorteilhaft ist es, wenn die mindestens eine Lagerelementaufnahme eine Länge aufweist, die mindestens doppelt so lang ist, wie eine Länge oder Breite einer Durchbrechung. Auf diese Weise wird eine Abschattung durch einen Boden oder eine Seitenwand des Siebkorbs für eine Reinigungsflüssigkeit in einer Spülmaschine weiter verringert.

Um eine Abschattung für eine Reinigungsflüssigkeit, insbesondere in einer Spülmaschine, möglichst gering zu halten, ist es vorteilhaft, wenn eine Breite einer Durchbrechung in einem Bereich von 2 mm bis 8 mm liegt.

Ferner ist es günstig, wenn eine Länge einer Durchbrechung in einem Bereich von 2 mm bis 15 mm liegt.

Um eine Abschattung durch Stege des Bodens und/oder einer Seitenwand zu minimieren, ist es vorteilhaft, wenn eine Breite der Stege in einem Bereich von 0,5 mm bis 4 mm liegt.

Vorzugsweise ist der Boden oder eine Seitenwand eben. Dies ermöglicht es, den Siebkorb aus einem Flachmaterial, beispielsweise einer Platte, herzustellen.

Gemäß einer bevorzugten Ausführungsform der Erfindung kann ferner vorgesehen sein, daß der Boden und/oder eine Seitenwand einstückig ausgebildet ist. Dadurch wird insbesondere die Stabilität des Siebkorbs erhöht. Insbesondere kann auch der Boden mit allen Seitenwänden einstückig verbunden sein, so daß die Seitenwände zusammen mit dem Boden perforiert und nach vollständiger Perforierung um eine Längs- oder Seitenkante des Bodens umgebogen werden können.

Vorteilhafterweise ist mindestens ein am Boden festlegbares Lagerelement zum Halten mindestens eines chirurgischen oder zahnchirurgischen Instruments oder Geräts vorgesehen. Mit dem Lagerelement kann sichergestellt werden, daß ein Instrument oder Gerät in einer definierten Stellung am Siebkorb gehalten wird. Insbesondere lassen sich so auch Beschädigungen von Instrumenten oder Geräten vermeiden, die aufgrund einer Relativbewegung zwischen Instrumenten oder Geräten, die direkt in den Siebkorb hineingelegt werden, entstehen können.

Vorzugsweise ist das mindestens eine Lagerelement mit dem Boden und/oder einer Seitenwand lösbar verbindbar. Damit kann es zum Reinigen des Siebkorbs auf einfache Weise entfernt werden. Zudem ist es möglich, das Lagerelement in jeweils gewünschter Weise in die eine oder eine andere Lagerelementaufnahme einzusetzen.

Besonders einfach läßt sich das mindestens eine Lagerelement mit dem Boden und/oder einer Seitenwand verbinden, wenn das mindestens eine Lagerelement mit dem Boden und/oder einer Seitenwand verrastbar oder am Boden und/oder der Seitenwand anclipbar ist. Alternativ wäre es auch denkbar, das Lagerelement mit dem Boden über einen Klemmsitz oder Preßsitz zu verbinden.

Vorteilhaft ist es, wenn das mindestens eine Lagerelement zum Festlegen am Boden mindestens ein mit einer Lagerelementaufnahme zusammenwirkendes Kupplungsglied aufweist. Das Lagerelement kann so zum Beispiel mit dem Boden oder einer Seitenwand verbunden werden, indem das Kupplungsglied mit dem Boden oder der Seitenwand verbunden wird, und zwar insbesondere mit einer Lagerelementaufnahme.

Eine besonders sichere Verbindung des Lagerelements mit dem Siebkorb wird erreicht dadurch, daß das mindestens eine Kupplungsglied in einer Lagerelementaufnahme einführbar ist. Es kann dann in dieser sicher gehalten werden. Beispielsweise kann das Kupplungsglied derart ausgebildet sein, daß es einen Hinterschnitt aufweist und so in der Lagerelementaufnahme gehalten wird.

Besonders einfach wird der Aufbau des Lagerelements, wenn das mindestens eine Kupplungsglied einen eine Lagerelementaufnahme durchsetzenden Kupplungssteg und mindestens ein am Steg angeordnetes Rückhalteelement umfaßt. Diese Ausgestaltung ermöglicht es, das Kupplungsglied in die Lagerelementaufnahme einzuführen, wobei das Rückhalteelement verhindert, daß das Kupplungsglied entgegen einer Einführrichtung wieder aus der Lagerelementaufnahme herausgezogen werden kann.

Ein besonders sicherer Halt wird gewährleistet, wenn das mindestens eine Rückhalteelement ein seitlich vom Kupplungssteg abstehender Vorsprung ist.

Das Verbinden des Lagerelements mit dem Siebkorb wird besonders einfach, wenn der Vorsprung in Form einer Rastnase ausgebildet ist, welche eine Aufgleitfläche aufweist, die beim Einführen des mindestens einen Kupplungsglied in eine Lagerelementaufnahme an dieser aufgleiten kann.

Um ein Spiel einer Verbindung zwischen dem Lagerelement und dem Siebkorb zu minimieren, ist es günstig, wenn der Kupplungssteg eine Höhe in einer Richtung quer zu einer vom Boden oder einer Seitenwand definierten Ebene aufweist und wenn die Höhe mindestens einer Dicke des Bodens oder der Seitenwand entspricht.

Gemäß einer bevorzugten Ausführungsform der Erfindung kann ferner vorgesehen sein, daß das mindestens eine Kupplungsglied einen vom mindestens einen Lagerelement abstehenden Vorsprung umfaßt, daß der Vorsprung seitliche Aufnahmen aufweist und daß eine Lagerelementaufnahme begrenzende Stege in die seitlichen Aufnahmen einführbar sind. Aufgrund dieser Ausgestaltung kann das Kupplungsglied in die Lagerelementaufnahme eingeführt werden, wobei es in der Lagerelementaufnahme dadurch gehalten wird, daß die Stege in die seitlichen Aufnahmen eintauchen und eine Relativbewegung zwischen dem Kupplungsglied und dem Siebkorb in einer Richtung quer zu einer vom Boden definierten Ebene verhindern.

Vorzugsweise entsprechen eine Länge und eine Breite des mindestens einen Kupplungsglieds in einer Ebene parallel zu der vom Boden oder einer Seitenwand definierten Ebene höchstens einer Länge und einer Breite einer Lagerelementaufnahme. Wird insbesondere die Abmessung einer Länge des Kupplungsglieds etwas kleiner gewählt als eine Länge der Lagerelementaufnahme, so können Fertigungstoleranzen bei der Herstellung sowohl der Lagerelemente als auch des Siebkorbs ausgeglichen werden.

Grundsätzlich wäre es denkbar, den Boden völlig eben zu belassen. Dies hat jedoch den Nachteil, daß eine Reinigungsflüssigkeit aus dem Siebkorb nur schlecht abfließen kann. Daher ist es vorteilhaft, wenn mindestens ein vom Boden auf einer Außenseite abstehender Fuß vorgesehen ist. Vorzugsweise sind mindestens drei, insbesondere jedoch vier Füße vorgesehen, die einen sicheren Stand des Siebkorbs auf einer Unterlage gewährleisten.

Grundsätzlich wäre es denkbar, den Fuß direkt am Boden anzuformen, beispielsweise durch Ausbilden einer Sicke oder durch Anschweißen. Vorteilhaft ist es jedoch, wenn mindestens ein Teil des mindestens einen Kupplungsglieds den mindestens einen Fuß bildet. Das Lagerelement übt somit eine Doppelfunktion aus, es dient zum einen zum Halten von Instrumenten oder Geräten, zum anderen dient es der Ausbildung eines Fußes, sodaß der Boden des Siebkorbs gegenüber einer Unterlage etwas angehoben ist, wodurch eine Reinigungsflüssigkeit besser aus dem Siebkorb ablaufen kann.

Günstig ist es, wenn der mindestens eine seitlich vom Kupplungssteg abstehende Vorsprung den mindestens einen Fuß bildet. Eine Höhe des Fußes und ein Abstand des Bodens von einer Unterlage, kann durch einen entsprechend ausgebildeten Vorsprung vorgegeben werden.

Gemäß einer bevorzugten Ausführungsform der Erfindung kann vorgesehen sein, daß das mindestens eine Lagerelement aus einem dampfsterilisierbaren Material hergestellt ist. So können im Siebkorb gehalterte Instrumente oder Geräte auch zum Sterilisieren im Siebkorb verbleiben.

Um Beschädigungen von Instrumenten oder Geräten zu minimieren oder gar ganz auszuschließen, ist es günstig, wenn das mindestens eine Lagerelement mindestens teilweise aus einem nachgiebigen oder elastischen Material hergestellt ist. Vorzugsweise ist das Material weich, beispielsweise ein Kunststoff.

Vorzugsweise ist das mindestens eine Kupplungsglied aus einem elastischen Material hergestellt. Dadurch kann das Lagerelement auf einfache Weise am Siebkorb angeclipst oder an diesem verrastet werden. Zudem kann der Siebkorb auf einer glatten und rutschigen Unterlage nicht wegrutschen, wenn das Kupplungsglied einen Fuß des Siebkorbs bildet.

Besonders eignet sich als nachgiebiges oder elastisches Material ein Kunststoff, vorzugsweise Silikon.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines erfindungsgemäßen Siebkorbs von oben;
- Figur 2:: eine perspektivische Ansicht des in Figur 1 dargestellten Siebkorbs von unten;
- Figur 3:: eine Draufsicht auf den in Figur 1 dargestellten Siebkorb;
- Figur 4:: eine Schnittansicht längs Linie 4-4 in Figur 3;
- Figur 5:: eine vergrößerte Ansicht des Bereichs A in Figur 3; und
- Figur 6:: eine vergrößerte Ansicht des Bereichs B in Figur 2.

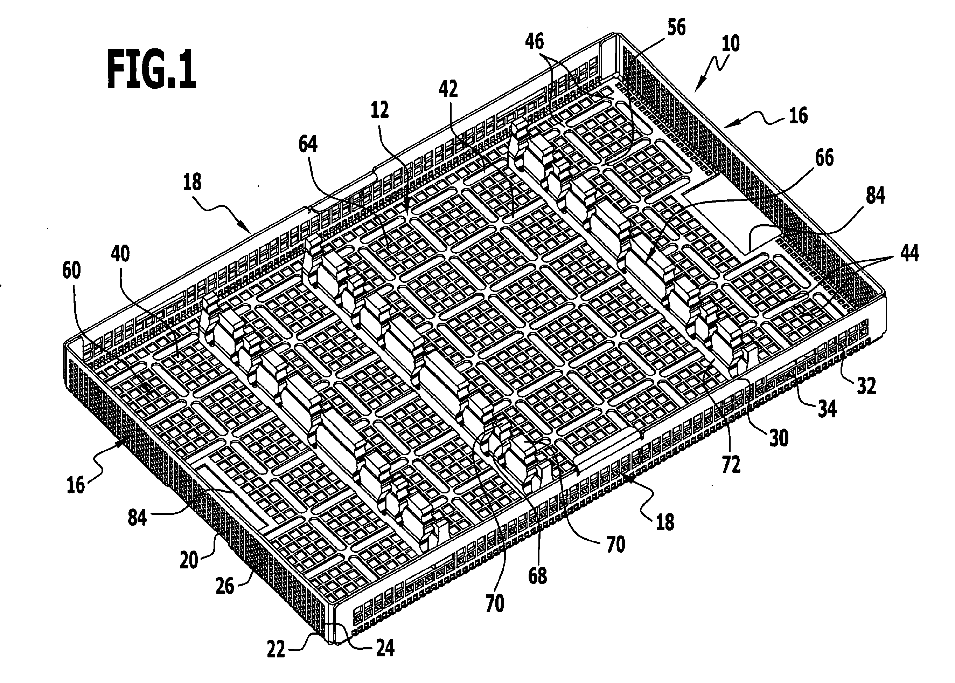

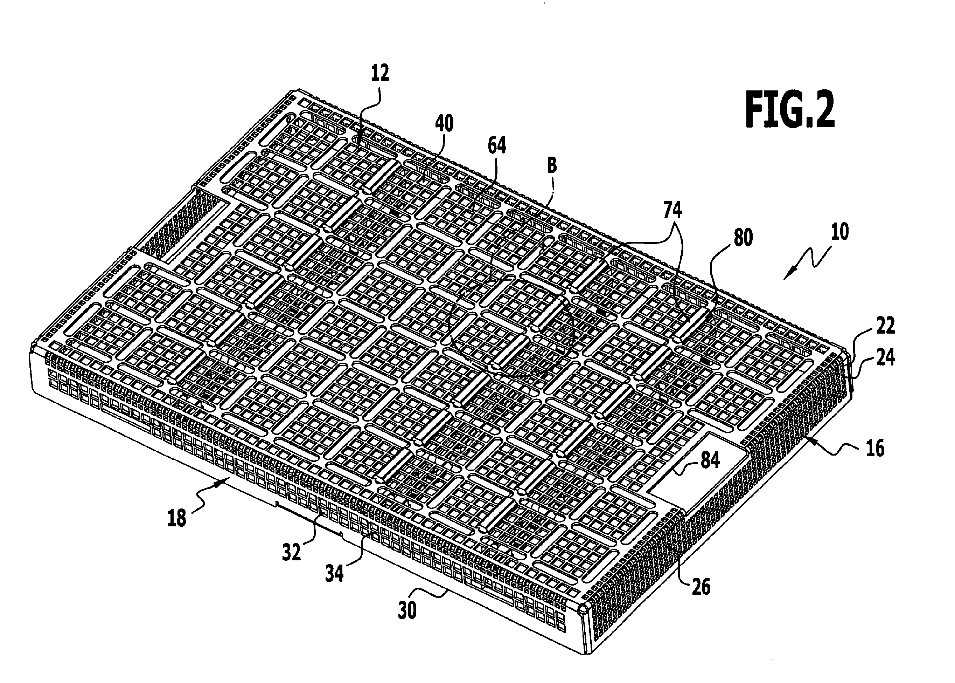

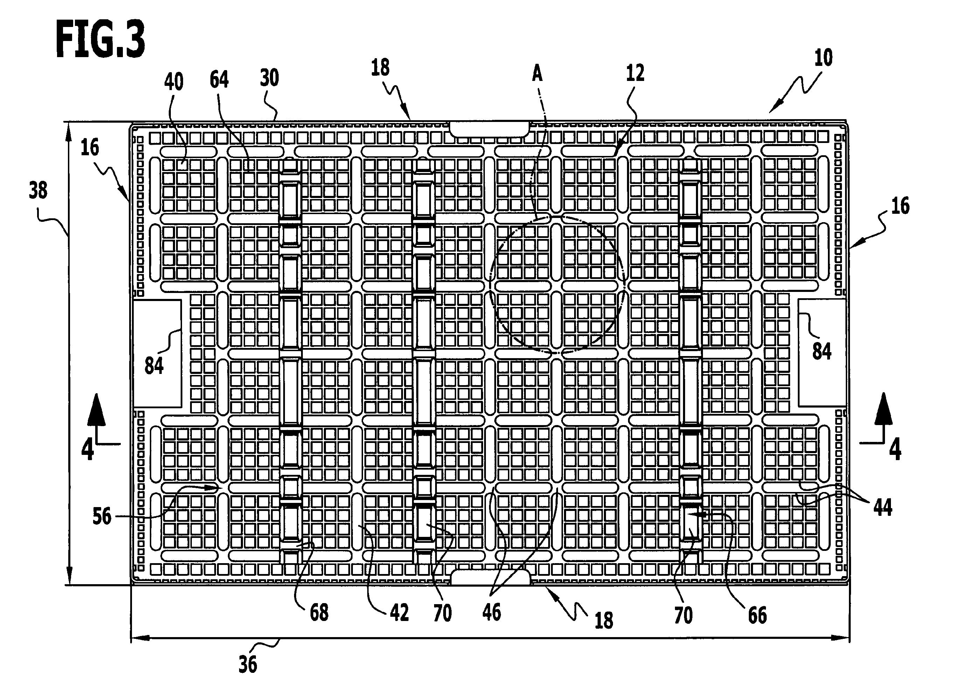

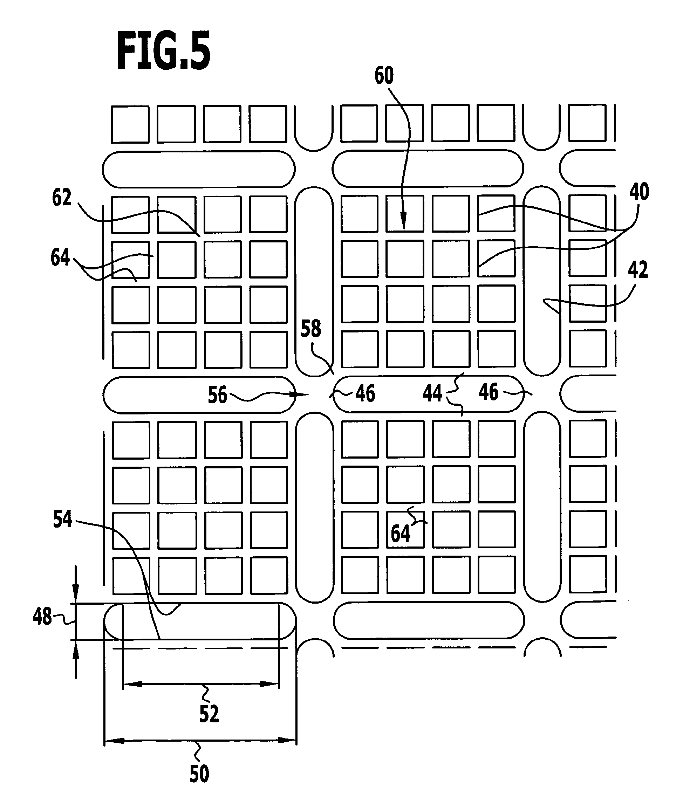

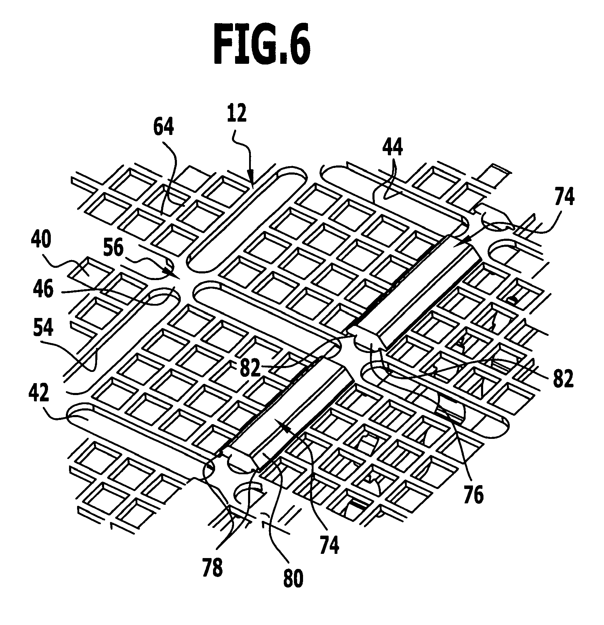

In den Figuren 1 bis 6 ist ein insgesamt mit dem Bezugszeichen 10 versehener Siebkorb dargestellt, welcher durch einen Boden 12 und insgesamt vier, jeweils paarweise parallel zueinander angeordneten Seitenwände 16 und 18 gebildet wird. Die Seitenwände 16, die Stirnseiten des Siebkorbs 10 bilden, sind mit einer Vielzahl von quadratischen Durchbrechungen 20 versehen, die in Reihen 22 und Spalten 24 parallel zu Seitenkanten der Seitenwände 16 angeordnet sind. Zwischen den Durchbrechungen verbleibende Stege 26 weisen eine Breite von 1 mm auf, die Durchbrechungen weisen Abmessungen von 2 x 2 mm auf.

Die Seitenwände 18 sind mit zwei parallel zueinander und zu einer Oberkante 30 der Seitenwände 18 verlaufenden Reihen von Durchbrechungen 32 versehen, die ebenfalls quadratisch sind, jedoch eine Größe von 4 x 4 mm aufweisen, wobei dazwischenliegende Stege wiederum eine Breite von 1 mm aufweisen.

Bei dem in den Figuren dargestellten Ausführungsbeispiel beträgt eine Länge 36 des Siebkorbs 10 269 mm, eine Breite 38 172 mm. Der Boden 12 ist ebenfalls mit einer Vielzahl von Durchbrechungen 40 versehen, die den Durchbrechungen 32 in Form und Größe entsprechen. Ferner sind Lagerelementaufnahmen bildende Langlöcher 42 vorgesehen, die durch zwei parallel zueinander verlaufende Längsstege 44 und zwei halbkreisförmig gekrümmte Querstege 46 begrenzt werden. Eine Breite 48 des Langlochs 42 beträgt 4 mm, eine Gesamtlänge 50 21 mm, eine Länge 52 paralleler Seitenkanten 54 des Langlochs 42 beträgt 17 mm. Ein Krümmungsradius der Querstege 46 beträgt 2 mm.

Jeweils vier Querstege 46 bilden ein Kreuzungselement 56, welches vier kreuzförmig mit ihren Längsrichtungen aufeinander zu orientierte Langlöcher 42 verbindet. Die Kreuzungselemente 56 verbinden jeweils Ecken 58 von vier quadratischen Flächenelementen 60, die außen begrenzt werden durch vier Längsstege 44. Jedes Flächenelement umfaßt 16 Durchbrechungen 40, die in vier Reihen und vier Spalten symmetrisch angeordnet sind. Sie bilden ein Durchbrechungsmuster mit einer vierzähligen Symmetrie.

Jedes Langloch 42 trennt zwei Längsstege 44 und damit zwei Flächenelemente 60 voneinander. Die Langlöcher 42 sind in parallel zu den Seitenwänden 16 und 18 verlaufenden Längs- und Querreihen angeordnet, die wiederum parallel zu Längs- und Querreihen der Flächenelemente 60 sind. Damit ergibt sich insgesamt eine Anordnung der Langlöcher 42 in Form eines Lagerelementaufnahmenmusters, welches ebenfalls eine vierzählige Symmetrie aufweist.

Mit anderen Worten kann das durch die Durchbrechungen 40 und die Langlöcher 42 insgesamt gebildete Muster auch so beschrieben werden. Ausgehend von in Längs- und Querreihen angeordneten Durchbrechungen 40, die den gesamten Boden 12 durchsetzen, sind in jeder fünften Reihe vier Durchbrechungen 40 zu einem Langloch 42 vereinigt. Die Kreuzungselemente 56 halten die durch die Langlöcher 42 separierten Flächenelemente 60 zusammen und sind an einer Stelle angeordnet, an der ursprünglich eine Durchbrechung 40 angeordnet gewesen wäre.

Jedes Kreuzungselement 56 weist einen Flächeninhalt auf, der in etwa sechzehnmal so groß ist wie eine Kreuzungsfläche 62, die definiert wird durch zwei sich kreuzende Stege 64, die die Durchbrechungen 40 der Flächenelemente 60 voneinander trennen. Eine Breite der Stege 64 entspricht einer Breite der Stege 44 und beträgt beim beschriebenen Ausführungsbeispiel 1 mm.

Zum Haltern von Instrumenten oder Geräten sind Lagerelemente 66 vorgesehen, welche hierfür entsprechende Einschnitte 68 aufweisen, die zwischen Abstandselementen 70 gebildet sind. In den Figuren 1 bis 3 sind jeweils drei Lagerelemente 66 am Siebkorb 10 angeordnet.

Die rein beispielhaft gezeichneten Lagerelemente 66 umfassen jeweils eine Leiste 72, von welcher die Abstandselemente 70 nach oben abstehen. Auf einer Unterseite der Leiste 72 sind langgestreckte, durch von der Leiste 72 abstehende Vorsprünge gebildete Kupplungsglieder 74 angeordnet, welche einen jeweils ein Langloch 42 durchsetzenden Kupplungssteg 76 und zwei von diesem seitlich abstehende Rastleisten 78 aufweisen, welche durch geneigte Außenflächen gebildete Aufgleitflächen 80 tragen. Die Lagerelemente 66 sind insgesamt einstückig aus Kunststoff, vorzugsweise Silikon, hergestellt, sodaß die Kupplungsglieder 74 mit den Aufgleitflächen 80 an die Längsstege 44 der Langlöcher 42 angelegt und aufgrund der Nachgiebigkeit beziehungsweise Elastizität der Rastleisten 78 sowie des Kupplungsstegs 76 durch das Langloch 42 hindurchgedrückt werden können. Die Rastleisten 78 federn nach dem Einführen der Kupplungsglieder 74 in die Langlöcher 42 seitlich aus und halten das Lagerelement 66 sicher am Boden 12. Die Kupplungsglieder 74 könnte man auch insgesamt als Vorsprung beschreiben, welcher parallel zueinander verlaufende seitliche Nuten 82 aufweist, in welche nach Einführen des Kupplungsglieds 74 in das Langlochs 42 die Längsstege 44 eintauchen und somit das Lagerelement 66 formschlüssig am Boden halten können.

Die in den Figuren 1 bis 3 dargestellten Lagerelemente 66 weisen eine Länge auf, die in etwa der Länge von sechs hintereinander angeordneten Langlöchern 42 entspricht. Sie umfassen jeweils sechs Kupplungsglieder 74, mit denen sie in sechs hintereinander angeordnete Langlöcher 42 eingeclipst werden können. Selbstverständlich können auch Lagerelemente am Boden 12 angeordnet werden, die nur ein Kupplungsglied aufweisen. Des weiteren lassen sich die Lagerelemente 66 oder auch kürzere, eine geringere Anzahl an Kupplungsgliedern 74 aufweisende Lagerelemente oder quadratische oder rechteckige, eine oder mehrere Flächenelemente 60 umgreifende Laserelemente mit entsprechenden Kupplungsgliedern parallel zu den Seitenwänden 18 am Boden 12 festlegen. Ausgehend von einem leeren, nur durch den Boden 12 und die Seitenwände 16 und 18 gebildeten Siebkorb 10 kann dieser individuell zur Aufnahme bestimmter Geräte und Instrumente durch Einbringen entsprechend geformter Lagerelemente vorbereitet werden.

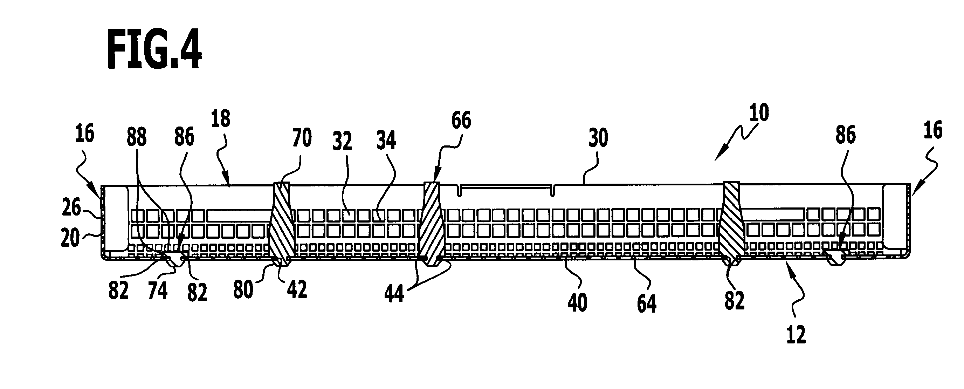

Die Kupplungsglieder 74 dienen zum einen dazu, die Lagerelemente 66 am Siebkorb 10 zu halten, zum anderen bilden die Kupplungsglieder 74, insbesondere die von einer Unterseite des Bodens 12 abstehenden Rastleisten 78, einen Fuß, auf welchem der Siebkorb 10 ruht. Ist das Lagerelement 66 aus einem Kunststoff hergestellt, so kann durch Wahl des entsprechenden Kunststoffmaterials verhindert werden, daß ein auf einer glatten Unterlage abgestellter Siebkorb 10 wegrutschen kann. Zudem stellt das vorspringende Kupplungsglied 74 sicher, daß Reinigungsflüssigkeit durch die Durchbrechungen 40 und die Langlöcher 42 besser nach unten ablaufen kann und sich nicht im Innern des Siebkorbs 10 sammelt. Anstelle der auch als Füße dienenden Lagerelemente 66 können auch separate Füße 86 vorgesehen werden, wie sie beispielhaft in Figur 4 eingezeichnet sind. Sie umfassen ein Kupplungsglied 74, zwei seitliche Nuten 82, in die beispielsweise Längsstege 44 oder Stege 64 eintauchen können, sodaß die Füße 86 insbesondere in den Langlöchern 42, bei entsprechend kleinen Abmessungen auch in eine oder mehrere Durchbrechungen 40 einclipsbar sind. Ein ins Innere des Siebkorbs 10 vorstehendes Element 88 dient als Gegenlager und zur seitlichen Begrenzungen der Nuten 82.

Durch die im Vergleich zu den Kreuzungsflächen 62 größere Fläche der Kreuzungselemente 56 können an diesen nicht dargestellte Lagerelemente auch dauerhaft befestigt werden, beispielsweise durch Punktschweißen, was aufgrund der geringen Fläche der Kreuzungsfläche 62 nicht oder nur sehr schwer möglich wäre.

Von den Seitenwänden 16 in den Boden 12 übergehend sind ferner rechteckige Aussparungen 84 vorgesehen, in die ein in einer nicht dargestellten Sterilcontainerwanne angeordneter Filterelementverschluß eintauchen kann.

Selbstverständlich können die Durchbrechungen 40 auch rechteckig geformt sein. Eine Symmetrie des Durchbrechungsmusters wäre dann nur noch zweizählig, das heißt bei Rotation des im Boden 12 vorgesehen Musters von Durchbrechungen 40 und Langlöchern 42 um eine zum Boden 12 senkrecht stehende Achse könnte das beziehungsweise die Muster nur in Deckung gebracht werden nach Drehung des Bodens um 180°.

## Patentansprüche

1. Siebkorb (10) zur Aufnahme chirurgischer oder zahnchirurgischer Instrumente oder Geräte mit einem Boden (12) und Seitenwänden (16, 18), wobei der Boden (12) und/oder die Seitenwände (16, 18) eine Vielzahl von Durchbrechungen (20, 32, 40) aufweisen, **dadurch gekennzeichnet, daß** der Boden (12) und/oder mindestens eine Seitenwand (16, 18) mindestens eine, eine von einer Form und/oder Größe der Durchbrechungen (40) abweichende Form und/oder Größe aufweisende Lagerelementaufnahme (42) zum Festlegen eines Lagerelements (66) zum Haltern mindestens eines chirurgischen oder zahnchirurgischen Instruments oder Geräts aufweist.

2. Siebkorb nach Anspruch 1, **dadurch gekennzeichnet, daß** die Durchbrechungen (40) in einem regelmäßigen Durchbrechungsmuster angeordnet sind.

3. Siebkorb nach Anspruch 2, **dadurch gekennzeichnet, daß** das Durchbrechungsmuster eine zwei-, drei-, vier- oder sechszählige Symmetrie aufweist.

4. Siebkorb nach einem der voranstehenden Ansprüche, **dadurch gekennzeichnet, daß** die Durchbrechungen (40) in Reihen und Spalten angeordnet sind.

5. Siebkorb nach einem der voranstehenden Ansprüche, **dadurch gekennzeichnet, daß** alle Durchbrechungen (40) identisch ausgebildet sind.

6. Siebkorb nach einem der voranstehenden Ansprüche, **dadurch gekennzeichnet, daß** die Durchbrechungen (40) eckig ausgebildet sind.

7. Siebkorb nach Anspruch 6, **dadurch gekennzeichnet, daß** die Durchbrechungen (40) rechteckig oder quadratisch ausgebildet sind.

8. Siebkorb nach einem der voranstehenden Ansprüche, **dadurch gekennzeichnet, daß** zwischen den Durchbrechungen (40) des Bodens (12) angeordnete Stege (64) vorgesehen sind.

9. Siebkorb nach Anspruch 8, **dadurch gekennzeichnet, daß** die Stege (64) drahtförmig ausgebildet und netzartig miteinander verbunden sind.

10. Siebkorb nach einem der Ansprüche 8 oder 9, **dadurch gekennzeichnet, daß** die Stege (64) einstückig ausgebildet sind als Streifen, die beim Einarbeiten der Durchbrechungen (40) in einen plattenförmigen Boden (12) zwischen den Durchbrechungen (40) stehenbleiben.

11. Siebkorb nach einem der voranstehenden Ansprüche, **dadurch gekennzeichnet, daß** mehrere in einem regelmäßigen Lagerelementaufnahmenmuster angeordnete Lagerelementaufnahmen (42) vorgesehen sind.

12. Siebkorb nach Anspruch 11, **dadurch gekennzeichnet, daß** das Lagerelementaufnahmenmuster eine zwei-, drei- vier- oder sechszählige Symmetrie aufweist.

13. Siebkorb nach einem der Ansprüche 11 oder 12, **dadurch gekennzeichnet, daß** das Lagerelementaufnahmenmuster ein das Durchbrechungsmuster unterbrechendes Muster ist, daß das Lagerelementaufnahmenmuster den Boden (12) und/oder eine Seitenwand (14, 16) in einzelne Flächenelemente (60) aufteilt und daß die Flächenelemente (60) nur Durchbrechungen (40) aufweisen.

14. Siebkorb nach Anspruch 13, **dadurch gekennzeichnet, daß** die Durchbrechungen (40) des Flächenelements (60) entsprechend dem Durchbrechungsmuster angeordnet sind.

15. Siebkorb nach einem der Ansprüche 13 oder 14, **dadurch gekennzeichnet, daß** eine Form der Flächenelemente (60) einer Form der Durchbrechungen (40) geometrisch ähnlich ist.

16. Siebkorb nach einem der Ansprüche 13 bis 15, **dadurch gekennzeichnet, daß** die Flächenelemente (60) regelmäßig angeordnet sind und daß die Flächenelemente (60) mehrere regelmäßig angeordnete Durchbrechungen (40) aufweisen.

17. Siebkorb nach einem der Ansprüche 13 bis 16, **dadurch gekennzeichnet, daß** die mindestens eine Lagerelementaufnahme (42) seitlich durch mindestens zwei Flächenelemente (60) begrenzt wird.

18. Siebkorb nach einem der Ansprüche 13 bis 17, **dadurch gekennzeichnet, daß** das mindestens eine Flächenelement (60) 16, jeweils in vier Reihen und Spalten angeordnete Durchbrechungen (40) aufweist.

19. Siebkorb nach einem der Ansprüche 13 bis 18, **dadurch gekennzeichnet, daß** alle Flächenelemente (60) identisch ausgebildet sind.

20. Siebkorb nach einem der Ansprüche 13 bis 19, **dadurch gekennzeichnet, daß** die Flächenelemente (60) über Kreuzungselemente (56) miteinander verbunden sind.

21. Siebkorb nach Anspruch 20, **dadurch gekennzeichnet, daß** die mindestens eine Lagerelementaufnahme (42) durch mindestens ein Kreuzungselement (56) begrenzt wird.

22. Siebkorb nach einem der Ansprüche 20 oder 21, **dadurch gekennzeichnet, daß** die mindestens eine Lagerelementaufnahme (42) von zwei Flächenelementen (60) und von zwei Kreuzungselementen (56) begrenzt wird.

23. Siebkorb nach einem der Ansprüche 20 bis 22, **dadurch gekennzeichnet, daß** das mindestens eine Kreuzungselement (56) Ecken (58) der Flächenelemente (60) miteinander verbindet.

24. Siebkorb nach Anspruch 23, **dadurch gekennzeichnet, daß** das mindestens eine Kreuzungselement (56) vier Flächenelemente (60) miteinander verbindet.

25. Siebkorb nach einem der Ansprüche 20 bis 24, **dadurch gekennzeichnet, daß** das mindestens eine Kreuzungselement (56) eine Fläche aufweist, die größer ist als eine von zwei sich kreuzenden, die Durchbrechungen (40) trennden Stege (64) gebildete Kreuzungsfläche (62).

26. Siebkorb nach Anspruch 25, **dadurch gekennzeichnet, daß** die vom mindestens einen Kreuzungselement (56) definierte Fläche mindestens zehnmal größer ist als die Kreuzungsfläche (62).

27. Siebkorb nach einem der Ansprüche 25 oder 26, **dadurch gekennzeichnet, daß** die vom mindestens einen Kreuzungselement (56) definierte Fläche in etwa einer Durchtrittsfläche einer Durchbrechung (40) entspricht.

28. Siebkorb nach einem der voranstehenden Ansprüche, **dadurch gekennzeichnet, daß** mindestens zwei Lagerelementaufnahmen (42) aneinander angrenzen.

29. Siebkorb nach einem der voranstehenden Ansprüche, **dadurch gekennzeichnet, daß** die mindestens eine Lagerelementaufnahme (42) in Form eines Langlochs ausgebildet ist.

30. Siebkorb nach einem der voranstehenden Ansprüche, **dadurch gekennzeichnet, daß** die mindestens eine Lagerelementaufnahme (42) von zwei parallel zueinander verlaufenden Längsstegen (44) und zwei die Längsstege (44) verbindenden Querstegen (46) begrenzt wird.

31. Siebkorb nach Anspruch 30, **dadurch gekennzeichnet, daß** die zwei Querstege (46) der mindestens einen Lagerelementaufnahme (42) aufeinander zu weisend konkav gekrümmt sind.

32. Siebkorb nach Anspruch 30 oder 31, **dadurch gekennzeichnet, daß** die Querstege (46) halbkreisförmig ausgebildet sind.

33. Siebkorb nach einem der Ansprüche 30 bis 32, **dadurch gekennzeichnet, daß** mindestens zwei Querstege (46) ein Kreuzungselement (56) bilden.

34. Siebkorb nach einem der voranstehenden Ansprüche, **dadurch gekennzeichnet, daß** die mindestens eine Lagerelementaufnahme (42) eine Länge (50, 52) aufweist, die mindestens doppelt so lang ist wie eine Länge oder Breite einer Durchbrechung (40).

35. Siebkorb nach einem der voranstehenden Ansprüche, **dadurch gekennzeichnet, daß** eine Breite einer Durchbrechung (40) in einem Bereich von 2 mm bis 8 mm liegt.

36. Siebkorb nach einem der voranstehenden Ansprüche, **dadurch gekennzeichnet, daß** eine Länge einer Durchbrechung (40) in einem Bereich von 2 mm bis 15 mm liegt.

37. Siebkorb nach einem der Ansprüche 8 bis 36, **dadurch gekennzeichnet, daß** eine Breite der Stege (26, 34, 44, 46, 64) in einem Bereich von 0,5 mm bis 4 mm liegt.

38. Siebkorb nach einem der voranstehenden Ansprüche, **dadurch gekennzeichnet, daß** der Boden (12) und/oder eine Seitenwand (16, 18) eben ist.

39. Siebkorb nach einem der voranstehenden Ansprüche, **dadurch gekennzeichnet, daß** der Boden (12) und/oder eine Seitenwand (16, 18) einstückig ausgebildet ist.

40. Siebkorb nach einem der voranstehenden Ansprüche, **dadurch gekennzeichnet, daß** mindestens ein am Boden (12) festlegbares Lagerelement (66) zum Haltern mindestens eines chirurgischen oder zahnchirurgischen Instruments oder Geräts vorgesehen ist.

41. Siebkorb nach Anspruch 40, **dadurch gekennzeichnet, daß** das mindestens eine Lagerelement (66) mit dem Boden (12) und/oder einer Seitenwand (16, 18) lösbar verbindbar ist.

42. Siebkorb nach einem der Ansprüche 40 oder 41, **dadurch gekennzeichnet, daß** das mindestens eine Lagerelement (66) mit dem Boden (12) und/oder einer Seitenwand (16, 18) verrastbar oder am Boden (12) und/oder einer Seitenwand (16, 18) anclipbar ist.

43. Siebkorb nach einem der Ansprüche 40 bis 42, **dadurch gekennzeichnet, daß** das mindestens eine Lagerelement (66) zum Festlegen am Boden (12) mindestens ein mit mindestens einer Lagerelementaufnahme (42) zusammenwirkendes Kupplungsglied (74) aufweist.

44. Siebkorb nach Anspruch 43, **dadurch gekennzeichnet, daß** das mindestens eine Kupplungsglied (74) in eine Lagerelementaufnahme (42) einführbar ist.

45. Siebkorb nach einem der Ansprüche 43 oder 44, **dadurch gekennzeichnet, daß** das mindestens eine Kupplungsglied (74) einen eine Lagerelementaufnahme (42) durchsetzenden Kupplungssteg (76) und mindestens ein am Kupplungssteg (76) angeordnetes Rückhalteelement (78) umfaßt.

46. Siebkorb nach Anspruch 45, **dadurch gekennzeichnet, daß** das mindestens eine Rückhalteelement (78) ein seitlich vom Kupplungssteg (76) abstehender Vorsprung ist.

47. Siebkorb nach Anspruch 46, **dadurch gekennzeichnet, daß** der Vorsprung (78) in Form einer Rastnase ausgebildet ist, welche eine Aufgleitfläche (80) aufweist, die beim Einführen des mindestens einen Kupplungsglieds (74) in eine Lagerelementaufnahme (42) an dieser aufgleiten kann.

48. Siebkorb nach einem der Ansprüche 45 bis 47, **dadurch gekennzeichnet, daß** der Kupplungssteg (76) eine Höhe in einer Richtung quer zu einer vom Boden (12) oder einer Seitenwand (16, 18) definierten Ebene aufweist und daß die Höhe mindestens einer Dicke des Bodens (12) oder der Seitenwand (16, 18) entspricht.

49. Siebkorb nach einem der Ansprüche 43 bis 48, **dadurch gekennzeichnet, daß** das mindestens eine Kupplungsglied (74) einen vom mindestens einen Lagerelement (66) abstehenden Vorsprung (78) umfaßt, daß der Vorsprung (78) seitliche Aufnahmen (82) aufweist und daß eine Lagerelementaufnahme (42) begrenzende Stege (44) in die seitlichen Aufnahmen (80) einführbar sind.

50. Siebkorb nach einem der Ansprüche 43 bis 49, **dadurch gekennzeichnet, daß** eine Länge und eine Breite des mindestens einen Kupplungsglieds (74) in einer Ebene parallel zu der vom Boden (12) oder einer Seitenwand (16, 18) definierten Ebene höchstens einer Länge (50, 52) und einer Breite (48) einer Lagerelementaufnahme (42) entsprechen.

51. Siebkorb nach einem der voranstehenden Ansprüche, **dadurch gekennzeichnet, daß** mindestens ein vom Boden (12) auf einer Außenseite abstehender Fuß (74, 86) vorgesehen ist.

52. Siebkorb nach Anspruch 51, **dadurch gekennzeichnet, daß** mindestens ein Teil des mindestens einen Kupplungsglieds (74) den mindestens einen Fuß (86) bildet.

53. Siebkorb nach einem der Ansprüche 51 oder 52, **dadurch gekennzeichnet, daß** der mindestens eine seitlich vom Kupplungssteg (76) abstehende Vorsprung (78) den mindestens einen Fuß (86) bildet.

54. Siebkorb nach einem der Ansprüche 40 bis 53, **dadurch gekennzeichnet, daß** das mindestens eine Lagerelement (66) aus einem dampfsterilisierbaren Material hergestellt ist.

55. Siebkorb nach einem der Ansprüche 40 bis 54, **dadurch gekennzeichnet, daß** das mindestens eine Lagerelement (66) mindestens teilweise aus einem nachgiebigen oder elastischen Material hergestellt ist.

56. Siebkorb nach Anspruch 55, **dadurch gekennzeichnet, daß** das mindestens eine Kupplungsglied (74) aus einem elastischen Material hergestellt ist.

57. Siebkorb nach einem der Ansprüche 55 oder 56, **dadurch gekennzeichnet, daß** das nachgiebige oder elastische Material Silikon ist.
